# EUROPÄISCHE PATENTANMELDUNG

(11) **EP 3 793 330 A1**
(43) Veröffentlichungstag der Anmeldung: **17.03.2021**
(21) Anmeldenummer: 19196954.2
(22) Anmeldetag: 12.09.2019
(51) Int. Cl.: H05G 1/02

(54) **RÖNTGENSTRAHLER**

(71) Anmelder: Siemens Healthcare GmbH, 91052 Erlangen (DE)
(72) Erfinder: Fritzler, Anja, 91052 Erlangen (DE); Havla, Lukas, 91301 Forchheim (DE); Röhrer, Peter, 91056 Erlangen (DE)

(57) **Zusammenfassung**

Die Erfindung betrifft einen Röntgenstrahler und einen Computertomographen.

Der erfindungsgemäße Röntgenstrahler (10) weist
- eine Röntgenröhre (11) und
- ein Röntgenstrahlergehäuse (G10) auf,
- wobei die Röntgenröhre ein evakuiertes Röntgenröhrengehäuse (G11), eine Kathode (K) zur Emission von Elektronen (E) und eine Anode (A) zur Generierung von Röntgenstrahlen in Abhängigkeit der Elektronen aufweist,
- wobei das Röntgenstrahlergehäuse die Röntgenröhre und außerhalb der Röntgenröhre ein gasförmiges Kühlmedium (L) aufweist, gekennzeichnet durch
- einen Verdichter (V) für eine erzwungene Konvektion des gasförmigen Kühlmediums zur Kühlung der Röntgenröhre, wobei ein Druckverhältnis zwischen Ansaugseite und Druckseite des Verdichters größer ist als 1,3.

## Beschreibung

Die Erfindung betrifft einen Röntgenstrahler und einen Computertomographen.

Üblicherweise weist ein Röntgenstrahler eine Röntgenröhre zur Generierung von Röntgenstrahlen auf, wofür insbesondere die Röntgenröhre gekühlt wird. Typischerweise wird für die Kühlung der Röntgenröhre ein flüssiges Kühlmedium verwendet, insbesondere Öl. Mit dem flüssigen Kühlmedium kann die Röntgenröhre aktiv oder passiv gekühlt werden. Bei der passiven Kühlung mit dem flüssigen Kühlmedium wird die Röntgenröhre insbesondere mittels Konvektion gekühlt. Bei der aktiven Kühlung mit dem flüssigen Kühlmedium wird typischerweise der Röntgenröhre ein abgekühltes flüssiges Kühlmedium zugeführt und ein erwärmtes flüssiges Kühlmedium entnommen.

Für die aktive Kühlung mit dem flüssigen Kühlmedium benötigt der Röntgenstrahler typischerweise einen Wärmetauscher zum Transferieren einer Wärmeenergie. Zusätzlich zum Wärmetauscher weist ein derartiger Röntgenstrahler typischerweise Schläuche und entsprechende Kupplungen auf, wodurch eine Komplexität und/oder ein Gewicht des Röntgenstrahlers üblicherweise signifikant erhöht ist.

Aus der DE 35 87 087 T2 ist ein Röntgenstrahler bekannt, bei dem das evakuierte Röntgenröhrengehäuse an einer Stirnseite eine Struktur aufweist, die Kanäle für ein flüssiges oder ein gasförmiges Kühlmedium bilden. An der Innenfläche dieser Struktur ist eine Anode derart angeordnet, dass die Rückseite der Anode durch das strömende Kühlmedium gekühlt wird.

Die US 4,355,410, die US 4,884,292 und die DE 698 25 248 T2 beschreiben jeweils ein Röntgenstrahlergehäuse, in dem eine Röntgenröhre und ein Ventilator angeordnet sind. Der Ventilator erzeugt eine Luftströmung, wodurch die Röntgenröhre während des Betriebs gekühlt wird. Gemäß der DE 698 25 248 T2 weist das evakuierte Röntgenröhrengehäuse der Röntgenröhre zur Verbesserung der Luftkühlung an seinem Außenumfang Kühlrippen auf.

In der US 6,134,299 ist eine Vorrichtung zur Erzeugung von Röntgenstrahlung offenbart, bei der eine Luftströmung um ein bzw. in einem Röntgenröhrengehäuse mittels Kühlrippen geführt ist.

Aus der WO 02/059932 A2 sowie der JP 2015032512 A ist jeweils ein Röntgenstrahler bekannt, der ein Röntgenstrahlergehäuse umfasst, in dem eine Röntgenröhre und ein Ventilator angeordnet sind. Während des Betriebs erzeugt ein Ventilator eine Luftströmung im Röntgenstrahlergehäuse, wodurch die Röntgenröhre von außen gekühlt wird.

Bei den vorstehend beschriebenen Röntgenstrahlern mit einem gasförmigen Kühlmedium (z.B. Luft) erfolgt die Zirkulation des gasförmigen Kühlmediums typischerweise durch einen Ventilator, der außerhalb des evakuierten Röntgenröhrengehäuses im Röntgenstrahlergehäuse angeordnet ist. Mit einem derartigen Ventilator ist nur eine geringe Wärmeabfuhr, insbesondere Kühlung, realisierbar, so dass - trotz der konstruktiv aufwändigen Lösung - für Röntgenstrahler nach wie vor die beschriebene Öl-Kühlung regelmäßig eingesetzt wird.

In der DE 298 23 735 U1 ist eine Drehkolben-Röntgenstrahler mit einem primären Kühlmittelkreislauf beschrieben, die einen externen Wärmetauscher umfasst. Im Kühlmittelkreislauf zirkuliert ein gasförmiges Kühlmittel, das gleichzeitig die Hochspannungsisolierung einer rotierenden Drehkolben-Röntgenröhre im Röntgenstrahlergehäuse übernimmt. Das Kühlmittel wird achsnah zugeführt und im anodenseitigen Bereich aus dem Röntgenstrahlergehäuse abgezogen. Die den Boden des rotierenden Drehkolbens bildende Anode ist vorzugsweise mit einer die Kühlfläche vergrößernden Profilierung versehen. Durch diese Profilierung wird eine Konvektion des gasförmigen Kühlmittels erzeugt. Diese Konvektion ist bestenfalls vergleichbar mit einer Konvektion, die mittels eines Ventilators erzielbar ist. Ein bevorzugtes Kühlmittel ist Schwefelhexafluorid.

Der Erfindung liegt die Aufgabe zu Grunde, einen Röntgenstrahler und einen Computertomographen anzugeben, bei welchen die Kühlung verbessert wird.

Die Aufgabe wird durch die Merkmale der unabhängigen Ansprüche gelöst. Vorteilhafte Ausgestaltungen sind in den Unteransprüchen beschrieben.

Der erfindungsgemäße Röntgenstrahler weist
- eine Röntgenröhre und
- ein Röntgenstrahlergehäuse auf,
- wobei die Röntgenröhre ein evakuiertes Röntgenröhrengehäuse, eine Kathode zur Emission von Elektronen und eine Anode zur Generierung von Röntgenstrahlen in Abhängigkeit der Elektronen aufweist,
- wobei das Röntgenstrahlergehäuse die Röntgenröhre und außerhalb der Röntgenröhre ein gasförmiges Kühlmedium aufweist, gekennzeichnet durch
- einen Verdichter für eine erzwungene Konvektion des gasförmigen Kühlmediums zur Kühlung der Röntgenröhre, wobei ein Druckverhältnis zwischen Ansaugseite und Druckseite des Verdichters größer ist als 1,3.

Ein Vorteil des Röntgenstrahler ist, dass die Kühlung des Röntgenstrahlers, insbesondere der Röntgenröhre, mit dem gasförmigen Kühlmedium eine Gewichtseinsparung von mehr als 10%, vorzugsweise mehr als 30%, im Vergleich zu einem mit einem flüssigen Kühlmedium gekühlten Röntgenstrahler ermöglicht. Typischerweise ist der Röntgenstrahler mit dem Verdichter leichter als ein herkömmlicher Röntgenstrahler. Ein weiterer Vorteil ist, dass eine Streuung der Röntgenstrahlen durch das gasförmige Kühlmedium wesentlicher geringer ist als eine Streuung der Röntgenstrahlen durch das flüssige Kühlmedium, wodurch vorzugsweise eine Bildqualität eines mittels der Röntgenstrahlen erfassten Bildes erhöht wird. Vorteilhafterweise fällt ein Entsorgungsproblem des flüssigen Kühlmediums dadurch weg, dass der Verdichter das gasförmige Kühlmedium zur Kühlung bereitstellt.

Der Röntgenstrahler kann in einem klinischen und/oder medizinischen Umfeld eingesetzt werden. Grundsätzlich ist es denkbar, dass der Röntgenstrahler für eine Materialprüfung und/oder für eine nicht-medizinische Durchleuchtung von Objekten außerhalb des klinischen und/oder medizinischen Umfelds, insbesondere in einem Sicherheitsumfeld eingesetzt wird.

Das Röntgenstrahlergehäuse weist üblicherweise Metall und/oder Glas auf und/oder ist im Wesentlichen zylinderförmig und/oder trichterförmig. Die Röntgenröhre, insbesondere das evakuierte Röntgenröhrengehäuse, ist typischerweise innerhalb des Röntgenstrahlergehäuses angeordnet. Typischerweise ist die Röntgenröhre nicht als eine Stehanoden-Röntgenröhre ausgebildet. Üblicherweise rotiert also wenigstens die Anode relativ zum Röntgenstrahlergehäuse.

Die Kathode kann einen Emitter zur Emission der Elektronen und/oder einen Fokuskopf aufweisen. Der Emitter kann ein Wendelemitter, ein Flachemitter, ein Feldeffekt-Emitter, ein direkt oder indirekt beheizbarer Emitter oder eine Kombination dergleichen sein.

Die Kathode und/oder die Anode sind typischerweise innerhalb des evakuierten Röntgenröhrengehäuses der Röntgenröhre angeordnet und/oder aufeinander ausgerichtet. Die Elektronen werden üblicherweise mit einer Beschleunigungsspannung von der Kathode auf die Anode zu beschleunigt. Die Beschleunigungsspannung ist typischer größer 10 kV und kleiner 200 kV, insbesondere zwischen 60 kV und 150 kV. Die Röntgenstrahlen werden üblicherweise bei einer Interaktion der beschleunigten Elektronen mit der Anode generiert. Die Anode weist typischerweise Wolfram, Gold, Rhenium, Rhodium, Molybdän und/oder eine Legierung der vorherigen Elemente auf. Grundsätzlich ist es denkbar, dass die Röntgenröhre den Fokuskopf und/oder eine elektromagnetische Ablenkeinheit zu einem Ablenken, insbesondere zu einem Fokussieren, der emittierten Elektronen aufweist. Üblicherweise wird weniger als 5% einer kinetischen Energie der beschleunigten Elektronen in Röntgenstrahlen umgewandelt, wobei die restliche Energie in Form von Hitze insbesondere an der Anode entsteht. Typischerweise muss die Anode am stärksten gekühlt werden.

Das Röntgenröhrengehäuse ist typischerweise luftdicht verschlossen. Während die Kathode und die Anode in dem evakuierten Röntgenröhrengehäuse angeordnet sind, ist typischerweise außerhalb der Röntgenröhre und innerhalb des Röntgenstrahlergehäuses das gasförmige Kühlmedium. Das gasförmige Kühlmedium weist üblicherweise Luft, insbesondere getrocknete Luft, Stickstoff, Sauerstoff, Neon, Kohlendioxid, Schwefelhexafluorid und/oder eine Mischung der vorherigen Elemente auf. Grundsätzlich ist es denkbar, dass das gasförmige Kühlmedium ausschließlich aus Luft besteht. Die Röntgenröhre, insbesondere das Röntgenröhrengehäuse und/oder die Anode und/oder die Kathode, werden vorzugsweise mittels des gasförmigen Kühlmediums gekühlt. Die Kühlung erfolgt insbesondere ohne ein flüssiges Kühlmedium. Mittels des Verdichters kann die Röntgenröhre vorzugsweise derart gekühlt werden, dass ein Dauerbetrieb des Röntgenstrahlers, beispielsweise im medizinischen Umfeld ermöglicht wird. Der Dauerbetrieb des Röntgenstrahlers umfasst beispielsweise mehrere konsekutive bildgebende Untersuchungen mit Röntgenstrahlen eines Patienten ohne Kühlpause und/oder konsekutive bildgebende Untersuchungen mit Röntgenstrahlen mehrerer Patienten ohne Kühlpause. Ein durch den Verdichter erwirkter Wärmeübergangskoeffizient innerhalb des Röntgenstrahlers beträgt insbesondere mindestens 300 W/(K^{∗}m^2), vorzugsweise mindestens 1000 W/(K^{∗}m^2), besonderes vorteilhafterweise mindestens 1500 W/(K^{∗}m^2).

Es ist denkbar, dass das Röntgenstrahlergehäuse insbesondere eine Zuluftöffnung und eine Abluftöffnung aufweist. Die Zuluftöffnung kann insbesondere auf der Ansaugseite des Verdichters sein. Die Abluftöffnung kann insbesondere auf der Druckseite des Verdichters sein. Die Kühlung erfolgt insbesondere durch die erzwungene Konvektion, wobei Wärme von der Röntgenröhre weg mittels des gasförmigen Kühlmediums übertragen wird. Das gasförmige Kühlmedium ist insbesondere an der Abluftöffnung wärmer als an der Zuluftöffnung. Das gasförmige Kühlmedium strömt insbesondere von der Zuluftöffnung zur Abluftöffnung.

Typischerweise korreliert der Wärmeübergangskoeffizient mit einem Druckverhältnis des Verdichters. Vorzugsweise ist auf der Druckseite des Verdichters der Druck um mindestens 0,5 Bar, vorzugsweise um 1 Bar, besonders vorteilhafterweise um 2 Bar oder 4 Bar höher als der Druck auf der Ansaugseite. Wenn auf der Ansaugseite des Verdichters ein atmosphärischer Druck von ca. 1 Bar ist und auf der Druckseite der Druck ca. 1,5 Bar ist, dann beträgt das Druckverhältnis 1,5. In diesem Fall ist also das Druckverhältnis des Verdichters größer 1,3. In einem weiteren Ausführungsbeispiel ist das Druckverhältnis größer als 2, vorzugsweise größer als 4, besonders vorteilhafterweise größer als 8. Das Druckverhältnis kann insbesondere an der Außenseite des Röntgenröhrengehäuses auf Höhe der Anode am größten sein.

Der Verdichter kann aktiv oder passiv sein. Der passive Verdichter weist beispielsweise eine passive Vorrichtung auf, welche insbesondere aufgrund der äußeren Beschaffenheit und/oder Form die Konvektion erzwingt. Der aktive Verdichter weist beispielsweise eine aktive Vorrichtung mit Verdichterelementen auf, welche Verdichterelemente insbesondere durch eine direkte oder indirekte Zufuhr von elektrischer oder mechanischer Leistung die Konvektion erzwingen. Der aktive Verdichter kann beispielsweise zusätzlich die passive Vorrichtung des passiven Verdichters aufweisen.

Eine Ausführungsform sieht vor, dass das Röntgenstrahlergehäuse und das Röntgenröhrengehäuse als turbinenförmiger Verdichter zum Erzwingen der Konvektion ausgebildet sind. Diese Ausführungsform kann insbesondere vorteilhaft sein, da die Konvektion vorzugsweise passiv erzwungen werden kann. Der turbinenförmige Verdichter ist in dieser Ausführungsform ein passiver Verdichter. Zum Erzwingen der Konvektion kann der turbinenförmige Verdichter insbesondere einen sich verjüngenden Querschnitt aufweisen und/oder trichterförmig sein. Eine Innenseite des Röntgenstrahlergehäuse und/oder eine Außenseite des Röntgenröhrengehäuse können eine Profilierung zum Erzwingen der Konvektion aufweisen. Insbesondere für einen Röntgenstrahler mit einer vergleichsweise geringen Leistung und entsprechender Wärmeentwicklung kann eine passiv erzwungene Konvektion ausreichend sein. Ein weiterer Vorteil dieser Ausführungsform kann sein, dass ein Verschleiß des Röntgenstrahlers im Vergleich zu einem herkömmlichen Röntgenstrahler mit einem Ventilator verringert ist. Ein Wärmeübergangskoeffizient dieser Ausführungsform ist insbesondere größer 300 W/(K^{∗}m^2), vorzugsweise größer 1500 W/(K^{∗}m^2).

Eine Ausführungsform sieht vor, dass die Anode sich mit einer relativ zum Röntgenstrahlergehäuse rotierenden Welle dreht, wobei der Verdichter mehrere Turbinenschaufeln zum Erzwingen der Konvektion aufweist und wobei die mehreren Turbinenschaufeln derart auf der rotierenden Welle gelagert sind, dass eine Drehgeschwindigkeit der Anode und eine Drehgeschwindigkeit der mehreren Turbinenschaufeln voneinander abhängen. Die Drehgeschwindigkeit der Anode korreliert insbesondere mit der Drehgeschwindigkeit der mehreren Turbinenschaufeln. Der Verdichter mit den mehreren sich drehenden Turbinenschaufeln ist insbesondere ein aktiver Verdichter. Diese Ausführungsform ist insbesondere vorteilhaft, wenn die mehreren Turbinenschaufeln und die Anode mittels der rotierenden Welle vorzugsweise fest verbunden, insbesondere mechanisch gekoppelt sind. Die mehreren Turbinenschaufeln drehen sich insbesondere mit der Anode und/oder mit der rotierenden Welle mit, typischerweise mit einer korrelierten oder mit derselben Drehgeschwindigkeit. Grundsätzlich ist es denkbar, dass die mehreren Turbinenschaufeln und die Anode über ein Getriebe mit einem Übersetzungsverhältnis insbesondere ungleich 1 gekoppelt sind, wodurch die Drehgeschwindigkeit der mehreren Turbinenschaufeln und die Drehgeschwindigkeit der Anode sich unterscheiden können, aber zumindest korrelieren. Die Drehgeschwindigkeit der mehreren Turbinenschaufeln und die Drehgeschwindigkeit der Anode können sich insbesondere entsprechen. Insbesondere wenn die Anode und die mehreren Turbinenschaufeln auf der rotierenden Welle, insbesondere ohne Getriebe, gelagert sind, kann die Drehgeschwindigkeit der Anode der Drehgeschwindigkeit der mehreren Turbinenschaufeln entsprechen. Diese Ausführungsform ist insbesondere vorteilhaft, weil ein Motor der Röntgenröhre für das Drehen der mehreren Turbinenschaufeln und der Anode verwendet werden kann. Die mehreren Turbinenschaufeln und die Anode werden insbesondere vom Motor der Röntgenröhre gleichzeitig angetrieben. Die mehreren Turbinenschaufeln können auf mehreren Turbinenschaufelebenen verteilt angeordnet sein. Grundsätzlich ist es denkbar, dass der Verdichter zusätzlich zu den sich drehenden Turbinenschaufeln statische Turbinenschaufeln aufweist, um die Konvektion zu erzwingen und/oder um die erzwungene Konvektion zu verstärken. Diese Ausführungsform kann insbesondere für einen Röntgenstrahler mit einer vergleichsweisen hohen Leistung und entsprechender Wärmeentwicklung vorteilhaft sein. Mittels der mehreren Turbinenschaufeln wird die Konvektion insbesondere aktiv erzwungen. Ein Wärmeübergangskoeffizient dieser Ausführungsform ist insbesondere größer 300 W/(K^{∗}m^2), vorzugsweise größer 1500 W/(K^{∗}m^2).

In einer bevorzugten Ausführungsform sind der turbinenförmige Verdichter und die mehreren Turbinenschaufeln der beiden vorher beschriebenen Ausführungsformen in dem Röntgenstrahler enthalten, was insbesondere für besonders leistungsfähige Röntgenstrahler mit entsprechender Wärmeentwicklung vorteilhaft sein kann. In diesem bevorzugten Ausführungsbeispiel ist also ein aktiver Verdichter enthalten. Der Röntgenstrahler ist in diesem Ausführungsbeispiel derart ausgebildet, dass das Röntgenstrahlergehäuse und das Röntgenröhrengehäuse als turbinenförmiger Verdichter zum Erzwingen der Konvektion ausgebildet sind und dass die Anode sich mit der relativ zum Röntgenstrahlergehäuse rotierenden Welle dreht, wobei der Verdichter die mehreren Turbinenschaufeln zum Erzwingen der Konvektion aufweist und wobei die mehreren Turbinenschaufeln derart auf der rotierenden Welle gelagert sind, dass die Drehgeschwindigkeit der Anode der Drehgeschwindigkeit des Verdichters entspricht. Ein Wärmeübergangskoeffizient dieser Ausführungsform ist insbesondere größer 300 W/(K^{∗}m^2), vorzugsweise größer 1500 W/(K^{∗}m^2).

Eine Ausführungsform sieht vor, dass die Röntgenröhre als eine Drehkolben-Röntgenröhre ausgebildet ist, wobei eine Drehgeschwindigkeit des Röntgenröhrengehäuses der Drehgeschwindigkeit der Anode entspricht. Vorteilhafterweise ist in der Drehkolben-Röntgenröhre lediglich ein Motor für das Rotieren der Anode und der Turbinenschaufeln und insbesondere für das Rotieren des Röntgenröhrengehäuses vorhanden. Diese Ausführungsform ist insbesondere vorteilhaft, weil bei der Drehkolben-Röntgenröhre das evakuierte Röntgenröhrengehäuse sich gemeinsam mit der Anode relativ zum Röntgenstrahlergehäuse dreht. Ein Drehwiderstand durch das gasförmige Kühlmedium ist im Vergleich zu einem Drehwiderstand durch ein herkömmliches flüssiges Kühlmedium vorzugsweise geringer. Die Drehkolben-Röntgenröhre kann sich vorzugsweise leichter drehen. Vorteilhafterweise kann eine Motorleistung der Drehkolben-Röntgenröhre daher geringer und/oder der zugehörige Motor günstiger sein. Ein weiterer Vorteil ist typischerweise, dass eine Peek-Hülle der Drehkolben-Röntgenröhre entfallen kann. Die Peek-Hülle weist üblicherweise Polyetheretherketon auf, welches insbesondere ein hochtemperetaturbeständiger thermoplastischer Kunststoff ist, und/oder ist üblicherweise in einem herkömmlichen Röntgenstrahler als zusätzliche Hülle des Röntgenröhrengehäuses drehbar gelagert, um eine Antriebsleistung der herkömmlichen Röntgenröhre zu verringern.

Eine zur Ausbildung als Drehkolben-Röntgenröhre alternative Ausführungsform sieht vor, dass die Röntgenröhre als eine Drehanoden-Röntgenröhre ausgebildet ist, wobei das Röntgenröhrengehäuse relativ zum Röntgenstrahlergehäuse ortsfest ist. In diesem Fall dreht sich das evakuierte Röntgenröhrengehäuse nicht relativ zum Röntgenstrahlergehäuse. Vorteilhafterweise ist in der Drehanoden-Röntgenröhre lediglich ein Motor für das Rotieren der Anode und der Turbinenschaufeln vorhanden.

Eine Ausführungsform sieht vor, dass das Röntgenröhrengehäuse ein Röntgenstrahlenaustrittfenster aufweist, wobei der Röntgenstrahler zusätzlich zum Verdichter eine Kühlplatte mit mehreren Löchern für eine Prallkühlung aufweist und wobei die Kühlplatte mit den mehreren Löchern relativ zum Röntgenstrahlenaustrittfenster derart ausgerichtet ist, dass ein durch die Kühlplatte in Abhängigkeit der erzwungenen Konvektion gerichteter gasförmiger Strom auf das Röntgenstrahlenaustrittfenster zur Prallkühlung auftrifft. Das Röntgenstrahlenaustrittfenster wird typischerweise von den Röntgenstrahlen zu einem Verlassen des Röntgenröhrengehäuses durchleuchtet, wobei das Röntgenstrahlenaustrittfenster sich typischerweise erhitzt. Diese Ausführungsform ermöglicht vorzugsweise eine ausreichende Kühlung des Röntgenstrahlenaustrittfensters mit dem gasförmigen Kühlmedium, insbesondere für konsekutive bildgebende Untersuchungen mit Röntgenstrahlung. Ein Wärmeübergangskoeffizient dieser Ausführungsform ist insbesondere größer 300 W/(K^{∗}m^2), vorzugsweise größer 1500 W/(K^{∗}m^2).

Eine Ausführungsform sieht vor, dass der Röntgenstrahler zusätzlich zum Verdichter eine Kühlplatte mit mehreren Nadeln für eine Pin-Fin-Kühlung aufweist und dass die mehreren Nadeln der Kühlplatte derart mit dem Röntgenröhrengehäuse befestigt sind, dass ein zwischen den Nadeln in Abhängigkeit der erzwungenen Konvektion gerichteter gasförmiger Strom auf das Röntgenröhrengehäuse zur Kühlung auftrifft. Die Kühlplatte kann insbesondere auf der Außenseite des Röntgenröhrengehäuses auf einer Höhe zwischen der Anode und der Kathode angeordnet sein. Ein Wärmeübergangskoeffizient dieser Ausführungsform ist insbesondere größer 300 W/(K^{∗}m^2), vorzugsweise größer 1500 W/(K^{∗}m^2).

Der erfindungsgemäße Computertomograph weist einen stationären Trägerring und einen rotierenden Trägerring auf, wobei der rotierende Trägerring den Röntgenstrahler und einen Röntgendetektor aufweist. Typischerweise werden mit den Röntgenstrahlen erzeugte Schwächungsprofile an dem Röntgendetektor erfasst, woraus ein röntgenstrahlenbasiertes Bild rekonstruiert werden kann. Das röntgenstrahlenbasierte Bild kann beispielsweise auf einer Anzeigeeinheit bereitgestellt und/oder in einer Speichereinheit abgespeichert werden. Vorteilhafterweise kann eine Motorleistung eines Motors des Computertomographen zum Bereitstellen einer Eigenrotation des rotierenden Teils verringert werden, weil der Röntgenstrahler anstatt mit dem herkömmlichen flüssigen Kühlmedium mit dem gasförmigen Kühlmedium gekühlt wird und typischerweise daher leichter ist. Vorzugsweise kann der rotierende Trägerring kleiner und/oder leichter und/oder weniger stabil ausgeführt werden, wenn der Röntgenstrahler leichter ist.

Eine Ausführungsform sieht vor, dass der Computertomograph derart ausgebildet ist, dass die durch den Verdichter des Röntgenstrahlers erzwungene Konvektion des gasförmigen Kühlmediums durch eine Eigenrotation des rotierenden Trägerrings verstärkt wird. Die Eigenrotation des rotierenden Trägerrings kann vorzugsweise einen Luftstrom auf der Ansaugseite des Verdichters derart bereitstellen, dass der Druck auf der Druckseite des Verdichters erhöht ist und/oder damit die Kühlung durch den Verdichter verstärkt wird. Die Eigenrotation des rotierenden Trägerring beschreibt insbesondere ein Rotieren des Trägerrings mit dem Röntgenstrahler um 360°. Eine Frequenz der Eigenrotation beträgt insbesondere mehr als 0,1 Hz, vorzugsweise mehr als 1 Hz, besonders vorteilshafterweise mehr als 5 Hz. Der Röntgenstrahler kann beispielsweise derart auf dem rotierenden Trägerring angeordnet sein, dass in Rotationsrichtung des Computertomographen die Ansaugseite des Verdichters und entgegen der Rotationsrichtung die Druckseite des Verdichters angeordnet ist. Alternativ oder zusätzlich kann der rotierende Trägerring und/oder der stationäre Trägerring eine Profilierung insbesondere auf einer radialen Außenseite aufweisen, um den Luftstrom bei der Eigenrotation zu erzeugen. Es ist denkbar, dass der rotierende Trägerring und/oder der stationäre Trägerring weitere Turbinenschaufeln insbesondere auf der radialen Außenseite aufweist, um den Luftstrom bei der Eigenrotation zu erzeugen.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 einen Röntgenstrahler 10 in einem ersten Ausführungsbeispiel,
Fig. 2 einen Röntgenstrahler 10 in einem zweiten Ausführungsbeispiel,
Fig. 3 einen Röntgenstrahler 10 in einem dritten Ausführungsbeispiel,
Fig. 4 einen Röntgenstrahler 10 in einem vierten Ausführungsbeispiel,
Fig. 5 einen Röntgenstrahler 10 in einem fünften Ausführungsbeispiel und
Fig. 6 einen Computertomograph 20 in einem sechsten Ausführungsbeispiel.

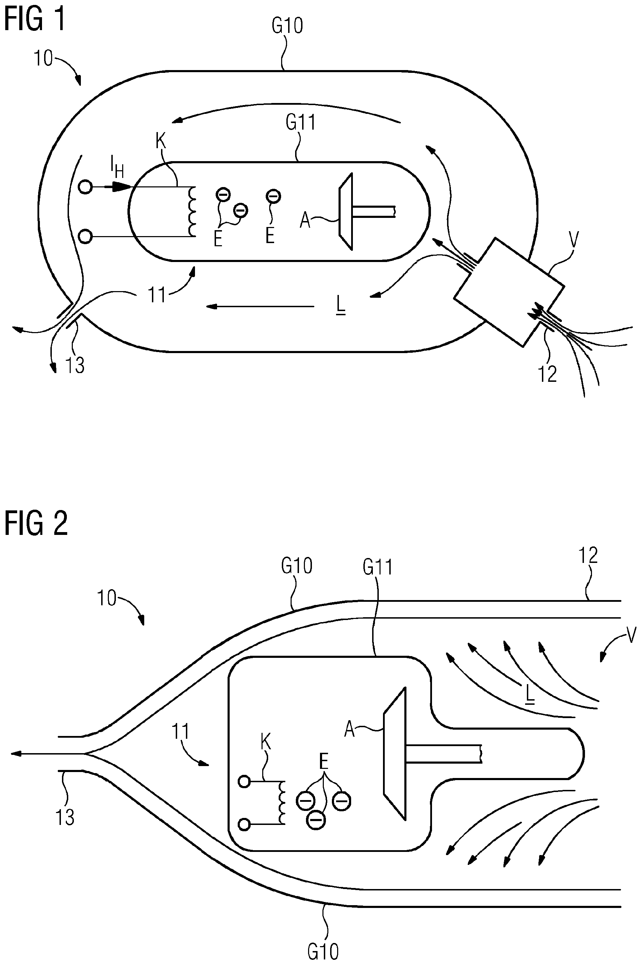

**Fig. 1** zeigt einen Röntgenstrahler 10 in einem ersten Ausführungsbeispiel. Der Röntgenstrahler 10 weist eine Röntgenröhre 11 und ein Röntgenstrahlergehäuse G10 auf. Die Röntgenröhre 11 weist ein evakuiertes Röntgenröhrengehäuse G11, eine Kathode K zur Emission von Elektronen E und eine Anode A zur Generierung von Röntgenstrahlen in Abhängigkeit der Elektronen E auf. Die Elektronen E werden beim Anlegen eines Heizstroms I_{H} von der Kathode K emittiert und von einer nicht gezeigten Beschleunigungsspannungsversorgung auf die Anode A zu beschleunigt.

Das Röntgenstrahlergehäuse G10 weist die Röntgenröhre 11 und außerhalb der Röntgenröhre 11 ein gasförmiges Kühlmedium L auf. Das gasförmige Kühlmedium L umströmt das Röntgenröhrengehäuse G11, um die Röntgenröhre 11 zu kühlen. Das gasförmige Kühlmedium L ist außerhalb des evakuierten Röntgenröhrengehäuses G11.

Der Röntgenstrahler 10 weist einen Verdichter V für eine erzwungene Konvektion des gasförmigen Kühlmediums L zur Kühlung der Röntgenröhre 11 auf. Ein Druckverhältnis zwischen Ansaugseite und Druckseite des Verdichters V ist größer als 1,3, in einem weiteren bevorzugten Ausführungsbeispiel größer als 2. Der Verdichter V ist in diesem Ausführungsbeispiel als Teil des Röntgenstrahlergehäuses G10 ausgebildet und bildet eine Zuluftöffnung 12. Eine Abluftöffnung 13 ist auf einer der Ansaugseite gegenüberliegenden Seite angeordnet. Die Druckseite des Verdichters V ist der Anode A zugewandt, während die Ansaugseite der Zuluftöffnung 12 zugewandt ist. Ein Druck innerhalb des Röntgenstrahlergehäuses G10 ist höher als ein Druck außerhalb des Röntgenstrahlergehäuses G10. Der in diesem Ausführungsbeispiel aktive Verdichter V kann beispielsweise einen aktiven Kompressor umfassen. Die in Fig. 1 dargestellten Pfeile zeigen schematisch einen Strömungsverlauf des gasförmigen Kühlmediums L. Das gasförmige Kühlmedium L umströmt bei der erzwungenen Konvektion die Röntgenröhre 11 zur Kühlung.

**Fig. 2** zeigt einen Röntgenstrahler 10 in einem zweiten Ausführungsbeispiel. Das Röntgenstrahlergehäuse G10 und das Röntgenröhrengehäuse G11 sind als turbinenförmiger Verdichter V zum Erzwingen der Konvektion ausgebildet. Der Röntgenstrahler 10 des zweiten Ausführungsbeispiels und der Röntgenstrahler 10 des ersten Ausführungsbeispiels unterscheiden sich insbesondere darin, dass in diesem Ausführungsbeispiel die Konvektion durch eine turbinenförmige Konstruktion des Röntgenstrahlergehäuses G10 und des Röntgenröhrengehäuses G11 vorzugsweise passiv erfolgt. Die in Fig. 2 dargestellten Pfeile zeigen schematisch einen Strömungsverlauf des gasförmigen Kühlmediums L. In diesem Ausführungsbeispiel ist ein Durchmesser der Zuluftöffnung 12 wesentlich größer als ein Durchmesser der Abluftöffnung 13. Das Röntgenstrahlergehäuse G10 und das Röntgenröhrengehäuse G11 wirken gemeinsam als Verdichter V aufgrund der turbinenförmigen und/oder trichterförmigen Form. Das Röntgenstrahlergehäuse G10 und das Röntgenröhrengehäuse G11 weisen jeweils einen sich verjüngenden Querschnitt auf, wobei in diesem Ausführungsbeispiel eine Ausrichtung antiparallel ist. Grundsätzlich ist denkbar, dass die Ausrichtung des Röntgenstrahlergehäuses G10 und des Röntgenröhrengehäuses G11 parallel ist. In diesem nicht gezeigten parallelen Fall zeigen Spitzen des Röntgenstrahlergehäuses G10 und des Röntgenröhrengehäuses G11 in dieselbe Richtung.

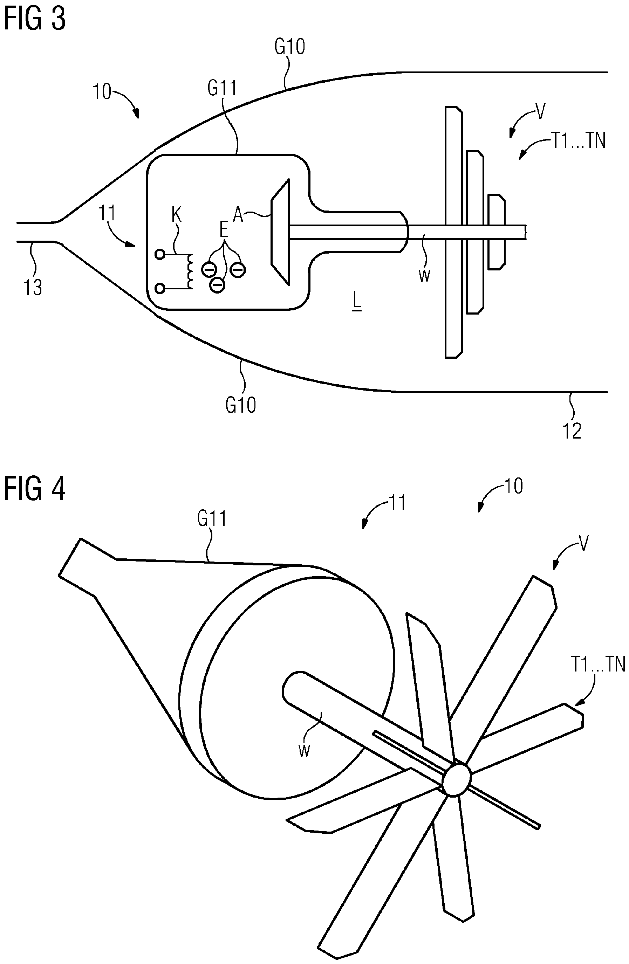

**Fig. 3** zeigt einen Röntgenstrahler 10 in einem dritten Ausführungsbeispiel. Dieses Ausführungsbeispiel baut auf dem zweiten Ausführungsbeispiel mit dem turbinenförmigen Verdichter V auf, wobei grundsätzlich eine andere Form des Röntgenstrahlergehäuses G10 und/oder des Röntgenröhrengehäuses G11 denkbar ist.

Die Anode A dreht sich mit einer relativ zum Röntgenstrahlergehäuse G10 rotierenden Welle W. Der Verdichter V weist mehrere Turbinenschaufeln T1...TN zum Erzwingen der Konvektion auf. Die mehreren Turbinenschaufeln T1...TN sind derart auf der rotierenden Welle W gelagert, dass eine Drehgeschwindigkeit der Anode A und eine Drehgeschwindigkeit des Verdichters V voneinander abhängen. Da die Anode A und die mehreren Turbinenschaufeln T1...TN direkt mit der rotierenden Welle W verbunden sind, entspricht in diesem Ausführungsbeispiel die Drehgeschwindigkeit der Anode A der Drehgeschwindigkeit des Verdichters V. Eine Drehachse der Anode entspricht einer Drehachse der Turbinenschaufeln T1...TN. Die mehreren, in diesem Ausführungsbeispiel 6, Turbinenschaufeln T1...TN sind in Fig. 3 in drei Turbinenschaufelebenen axial versetzt angeordnet. Typischerweise ist ein Winkel in einem axialen Schnitt zwischen den jeweiligen Turbinenschaufeln T1...TN gleich. Die Turbinenschaufeln T1...TN können in einem Anstellwinkel kleiner 90° auf der rotierenden Welle W angeordnet sein. Der insbesondere aktive Verdichter V weist beispielsweise mehr als 1, vorzugsweise 2 bis 36, besonders vorteilhafterweise 20 bis 26 Turbinenschaufeln T1...TN auf.

Dieses Ausführungsbeispiel kann grundsätzlich einer Ausführungsform entsprechen, wobei die Röntgenröhre 11 als eine Drehanoden-Röntgenröhre ausgebildet ist und wobei das Röntgenröhrengehäuse G11 relativ zum Röntgenstrahlergehäuse G12 ortsfest ist. In diesem Fall drehen sich insbesondere die Anode A und die mehreren Turbinenschaufeln T1...TN relativ zum Röntgenstrahlergehäuse G10. Das Röntgenröhrengehäuse G11 und das Röntgenstrahlergehäuse G11 ist derart ortsfest, dass sie nicht zueinander verdreht werden können.

**Fig. 4** zeigt einen Röntgenstrahler 10 in einem vierten Ausführungsbeispiel in einer perspektivischen Ansicht. Fig. 4 zeigt eine Weiterbildung des dritten Ausführungsbeispiels aus Gründen der Übersichtlichkeit ohne das Röntgenstrahlergehäuse G10. Fig. 4 zeigt acht radial um die Welle W in einer Turbinenschaufelebene angeordnete Turbinenschaufeln T1...TN.

In diesem Ausführungsbeispiel ist die Röntgenröhre 11 als eine Drehkolben-Röntgenröhre ausgebildet, wobei eine Drehgeschwindigkeit des Röntgenröhrengehäuses G11 der Drehgeschwindigkeit der Anode A entspricht. Die Anode A dreht sich gemeinsam mit den Turbinenschaufeln T1...TN und mit dem Röntgenröhrengehäuse G11 um eine gemeinsame Drehachse, beispielsweise die Drehachse der Anode A. Typischerweise ist die nicht in Fig. 4 gezeigte Kathode K auf der gemeinsamen Drehachse angeordnet und die emittierten Elektronen E werden mittels einer elektromagnetischen Ablenkeinheit in einen Randbereich der Anode A abgelenkt. Die in Fig. 4 gezeigten Drehkolben-Röntgenröhre ist im Wesentlichen kegelförmig. Grundsätzlich ist es denkbar, dass eine Drehkolben-Röntgenröhre im Wesentlichen doppelkegelförmig ist.

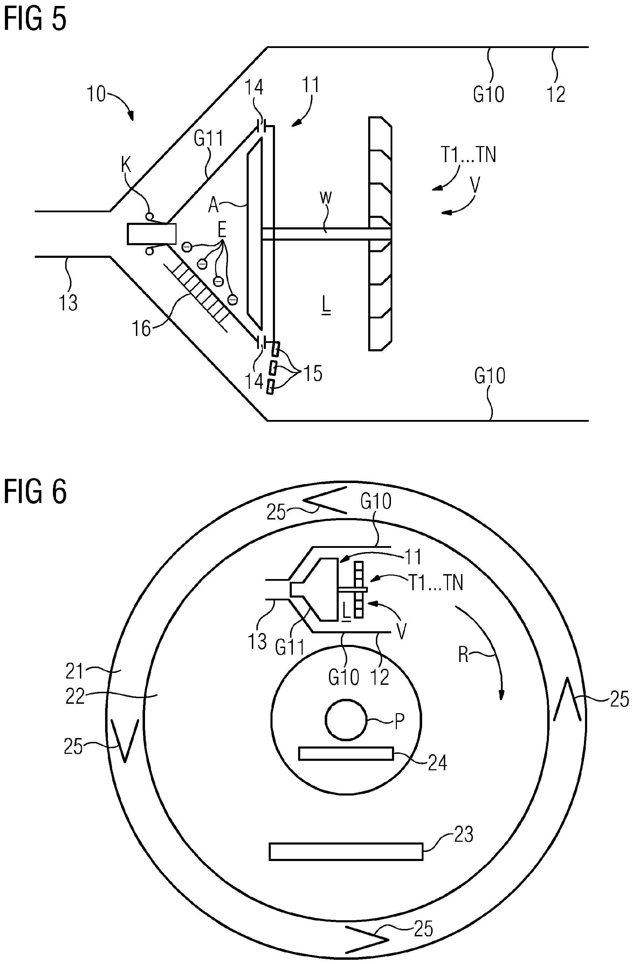

**Fig. 5** zeigt einen Röntgenstrahler 10 in einem vierten Ausführungsbeispiel. Fig. 5 zeigt eine Weiterbildung des in Fig. 4 gezeigten Ausführungsbeispiels.

Das Röntgenröhrengehäuse G11 weist ein Röntgenstrahlenaustrittfenster 14 auf. Der Röntgenstrahler 10 weist zusätzlich zum Verdichter V eine Kühlplatte 15 mit mehreren Löchern für eine Prallkühlung auf. Die Kühlplatte 15 mit den mehreren Löchern ist relativ zum Röntgenstrahlenaustrittfenster 14 derart ausgerichtet, dass ein durch die Kühlplatte 15 in Abhängigkeit der erzwungenen Konvektion gerichteter gasförmiger Strom auf das Röntgenstrahlenaustrittfenster 14 zur Prallkühlung auftrifft. Das Röntgenstrahlenaustrittfenster 14 weist typischerweise Glas, Titan, Beryllium, Aluminium, Stahl und/oder eine Kombination dieser Elemente auf. Das Röntgenstrahlenaustrittfenster 14 ist ringförmig ausgebildet. Das Röntgenröhrengehäuse G1 und das Röntgenstrahlenaustrittfenster 14 sind derart ausgebildet, dass das umschlossene Vakuum erhalten bleibt. Die Kühlplatte 15 mit den mehreren Löchern ist in Fig. 5 in einem Querschnitt und daher mit Unterbrechungen bei den Löchern gezeigt. Typischerweise umschließt die Kühlplatte 15 die mehreren Löcher. Die Kühlplatte 15 weist typischerweise Metall, insbesondere Aluminium und/oder Kupfer, Kunststoff, Polyetheretherketon, kohlenstoffverstärkte Fasern, ein stabiles Material und/oder eine Kombination dieser Materialien und/oder mehr als 2 Löcher auf. Ein Durchmesser zumindest eines Loches der mehreren Löcher der Kühlplatte 15 liegt beispielsweise zwischen 0,1 mm und 40 mm, vorzugsweise 1 mm und 4 mm, besonders vorteilhafterweise zwischen 2 mm und 3 mm. Die Kühlplatte 15 kann rotationsymmetrisch sein. Die Kühlplatte 15 mit den mehreren Löchern ist derart angeordnet, dass zumindest die meisten Röntgenstrahlen die Kühlplatte 15 verpassen, insbesondere nicht auf der Kühlplatte 15 auftreffen. Vorzugsweise ist der Röntgenstrahler 10 derart ausgebildet, dass die Röntgenstrahlen außerhalb des Röntgenstrahlergehäuses G10 erfasst werden können und nicht innerhalb des Röntgenstrahlergehäuses G10 vollständig absorbiert werden.

In diesem Ausführungsbeispiel weist der Röntgenstrahler 10 zusätzlich zum Verdichter V eine Kühlplatte 16 mit mehreren Nadeln für eine Pin-Fin-Kühlung auf. Die mehreren Nadeln der Kühlplatte 16 sind derart mit dem Röntgenröhrengehäuse G11 befestigt, dass ein zwischen den Nadeln in Abhängigkeit der erzwungenen Konvektion gerichteter gasförmiger Strom auf das Röntgenröhrengehäuse G11 zur Kühlung auftrifft. Die Kühlplatte 16 ist im Bereich des sich verjüngenden Röntgenröhrengehäuses G11 angeordnet. Die Kühlplatte 16 kann rotationssymmetrisch sein. Die Kühlplatte 16 ist derart auf der Druckseite des Verdichters V ausgerichtet, dass das gasförmige Kühlmedium mit dem erhöhten Druck zwischen der Kühlplatte 16 und der Außenseite des Röntgenröhrengehäuses G11 hindurch strömt. Die Kühlplatte 16 mit den mehreren Nadeln weist üblicherweise Metall, insbesondere Aluminium und/oder Kupfer, Kunststoff, Polyetheretherketon, kohlenstoffverstärkte Fasern, ein stabiles Material und/oder eine Kombination dieser Materialien und/oder mehr als 1 Nadel auf. Ein Durchmesser zumindest einer Nadel der mehreren Nadeln der Kühlplatte 16 liegt beispielsweise zwischen 0,1 mm und 10 mm, vorzugsweise zwischen 0,5 mm und 2 mm, besonders vorteilhafterweise zwischen 1 mm und 1,5 mm. Eine Länge der zumindest einen Nadel der mehreren Nadeln der Kühlplatte 16 liegt beispielsweise zwischen 0,1 mm und 50 mm, vorzugsweise zwischen 0,5 mm und 5 mm, besonders vorteilhafterweise zwischen 2 mm und 4 mm.

Grundsätzlich ist es denkbar, dass die Kühlplatte 15 mit den mehreren Löchern und/oder die Kühlplatte 16 mit den mehreren Nadeln an einer Seite des Röntgenröhrengehäuses G11 in einer Nähe zur Anode A angeordnet sind.

**Fig. 6** zeigt einen Computertomograph 20 in einem sechsten Ausführungsbeispiel. Der Computertomograph 20 weist einen stationären Trägerring 21 und einen rotierenden Trägerring 22 auf. Der stationäre Trägerring 21 und der rotierende Trägerring 22 weisen beispielsweise Metall und/oder Kunststoff auf und sind typischerweise rotationssymmetrisch. Der Computertomograph zeigt den rotierenden Trägerring 22 ohne Computertomographgehäuse.

Der rotierende Trägerring 22 weist einen Röntgenstrahler 10 und einen Röntgendetektor 23 auf. Zwischen dem Röntgenstrahler 10 und dem Röntgendetektor 23 ist ein Patient P auf einer Patientenliege 24 gelagert. Alternativ zum Patient P kann ein Gegenstand, beispielsweise ein Koffer, mit den Röntgenstrahlen durchleuchtet werden.

In diesem Ausführungsbeispiel ist der Computertomograph 20 derart ausgebildet, dass die durch den Verdichter V des Röntgenstrahlers 10 erzwungene Konvektion des gasförmigen Kühlmediums L durch eine Eigenrotation des rotierenden Trägerrings 22 verstärkt wird. Dafür ist die Zuluftöffnung 12 derart ausgerichtet, dass das gasförmige Kühlmedium L bei der Eigenrotation des rotierenden Trägerrings 22 in das Röntgenstrahlergehäuse G10 geführt und/oder gepresst wird. In diesem Ausführungsbeispiel weist der stationäre Trägerring 21 eine optionale Profilierung 25 auf, um die erzwungene Konvektion zusätzlich zu verstärken. Alternativ oder zusätzlich kann der rotierende Trägerring eine Profilierung aufweisen. Die Profilierung 25 kann insbesondere derart ausgebildet sein, dass das gasförmige Kühlmedium L in das Röntgenstrahlergehäuse G10 geführt und/oder gepresst wird. Die Profilierung 25 kann insbesondere weitere Turbinenschaufeln aufweisen und/oder wellenförmig und/oder schneckenförmig sen.

Aufgrund der mehreren Turbinenschaufeln T1...TN des Verdichters V ist der Verdichter ein aktiver Verdichter. Falls die mehreren Turbinenschaufeln T1...TN durch statische Turbinenschaufeln ersetzt werden, kann der passive Verdichter für eine Kühlung des Röntgenstrahlers 10 ausgebildet sein, insbesondere wenn die durch den Verdichter V des Röntgenstrahlers 10 erzwungene Konvektion des gasförmigen Kühlmediums L durch die Eigenrotation des rotierenden Trägerrings 22 verstärkt wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgenstrahler (10), aufweisend
- eine Röntgenröhre (11) und
- ein Röntgenstrahlergehäuse (G10),
- wobei die Röntgenröhre (11) ein evakuiertes Röntgenröhrengehäuse (G11), eine Kathode (K) zur Emission von Elektronen (E) und eine Anode (A) zur Generierung von Röntgenstrahlen in Abhängigkeit der Elektronen (E) aufweist,
- wobei das Röntgenstrahlergehäuse (G10) die Röntgenröhre (11) und außerhalb der Röntgenröhre (11) ein gasförmiges Kühlmedium (L) aufweist,
**gekennzeichnet durch**
- einen Verdichter (V) für eine erzwungene Konvektion des gasförmigen Kühlmediums (L) zur Kühlung der Röntgenröhre (11), wobei ein Druckverhältnis zwischen Ansaugseite und Druckseite des Verdichters (V) größer ist als 1,3.

2. Röntgenstrahler (10) nach Anspruch 1, wobei das Röntgenstrahlergehäuse (G10) und das Röntgenröhrengehäuse (G11) als turbinenförmiger Verdichter (V) zum Erzwingen der Konvektion ausgebildet sind.

3. Röntgenstrahler (10) nach einem der vorhergehenden Ansprüche, wobei die Anode (A) sich mit einer relativ zum Röntgenstrahlergehäuse (G10) rotierenden Welle (W) dreht, wobei der Verdichter (V) mehrere Turbinenschaufeln (T1...TN) zum Erzwingen der Konvektion aufweist und wobei die mehreren Turbinenschaufeln (T1...TN) derart auf der rotierenden Welle (W) gelagert sind, dass eine Drehgeschwindigkeit der Anode (A) und eine Drehgeschwindigkeit des Verdichters (V) voneinander abhängen.

4. Röntgenstrahler (10) nach Anspruch 3, wobei die Röntgenröhre (11) als eine Drehkolben-Röntgenröhre ausgebildet ist, wobei eine Drehgeschwindigkeit des Röntgenröhrengehäuses (G11) der Drehgeschwindigkeit der Anode (A) entspricht.

5. Röntgenstrahler (10) nach Anspruch 3, wobei die Röntgenröhre (11) als eine Drehanoden-Röntgenröhre ausgebildet ist, wobei das Röntgenröhrengehäuse (G11) relativ zum Röntgenstrahlergehäuse (G12) ortsfest ist.

6. Röntgenstrahler (10) nach einem der vorhergehenden Ansprüche, wobei das Druckverhältnis größer ist als 2.

7. Röntgenstrahler (10) nach einem der vorhergehenden Ansprüche, wobei das Röntgenröhrengehäuse (G10) ein Röntgenstrahlenaustrittfenster (14) aufweist, wobei der Röntgenstrahler (10) zusätzlich zum Verdichter (V) eine Kühlplatte (15) mit mehreren Löchern für eine Prallkühlung aufweist und wobei die Kühlplatte (15) mit den mehreren Löchern relativ zum Röntgenstrahlenaustrittfenster (14) derart ausgerichtet ist, dass ein durch die Kühlplatte (15) in Abhängigkeit der erzwungenen Konvektion gerichteter gasförmiger Strom auf das Röntgenstrahlenaustrittfenster (14) zur Prallkühlung auftrifft.

8. Röntgenstrahler (10) nach einem der vorhergehenden Ansprüche, wobei der Röntgenstrahler (10) zusätzlich zum Verdichter (V) eine Kühlplatte (16) mit mehreren Nadeln für eine Pin-Fin-Kühlung aufweist und wobei die mehreren Nadeln der Kühlplatte (16) derart mit dem Röntgenröhrengehäuse (G11) befestigt sind, dass ein zwischen den Nadeln in Abhängigkeit der erzwungenen Konvektion gerichteter gasförmiger Strom auf das Röntgenröhrengehäuse (G11) zur Kühlung auftrifft.

9. Computertomograph (20), aufweisend
- einen stationären Trägerring (21) und einen rotierenden Trägerring (22), wobei der rotierende Trägerring (22)
- einen Röntgenstrahler (10) nach einem der vorhergehenden Ansprüche und
- einen Röntgendetektor (23) aufweist.

10. Computertomograph (20) nach Anspruch 9, wobei der Computertomograph (20) derart ausgebildet ist, dass die durch den Verdichter (V) des Röntgenstrahlers (10) erzwungene Konvektion des gasförmigen Kühlmediums (L) durch eine Eigenrotation des rotierenden Trägerrings (22) verstärkt wird.
